# EUROPEAN PATENT APPLICATION

(11) **EP 4 295 863 A1**
(43) Date of publication of application: **27.12.2023**
(21) Application number: 21926648.3
(22) Date of filing: 04.03.2021
(51) Int. Cl.: A61K 39/395, A61P 29/00, A61P 37/06, C07K 16/00

(54) **ANTI-INFLAMMATORY, NON-FUCOSYLATED IMMUNOGLOBULIN PREPARATION AND PRODUCTION METHOD THEREFOR**

(30) Priority: 19.02.2021 JP 2021024790
(71) Applicant: Mimura, Yusuke, Ube-shi, Yamaguchi, 755-0241 (JP); Mimura, Yuka, Ube-shi, Yamaguchi, 755-0241 (JP)
(72) Inventor: Mimura, Yusuke, Ube-shi, Yamaguchi, 755-0241 (JP); Mimura, Yuka, Ube-shi, Yamaguchi, 755-0241 (JP)
(74) Representative: Elkington and Fife LLP
(86) International application number: PCT/JP2021/008404
(87) International publication number: WO 2022/176219

(57) **Abstract**

Provided is a novel therapeutic agent for inflammatory diseases such as autoimmune diseases. According to the present invention, an included IgG antibody comprises a human serum IgG antibody in which the sugar chain shown below is bonded to asparagine 297 (Asn297) in an Fc portion.

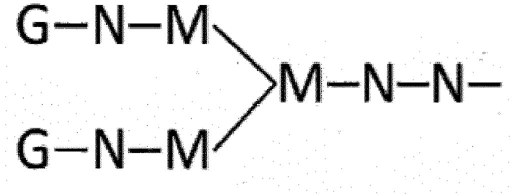

## Description

### Field of Art

The present invention relates to an anti-inflammatory afucosylated immunoglobulin preparation and a method of producing such.

### Background Art

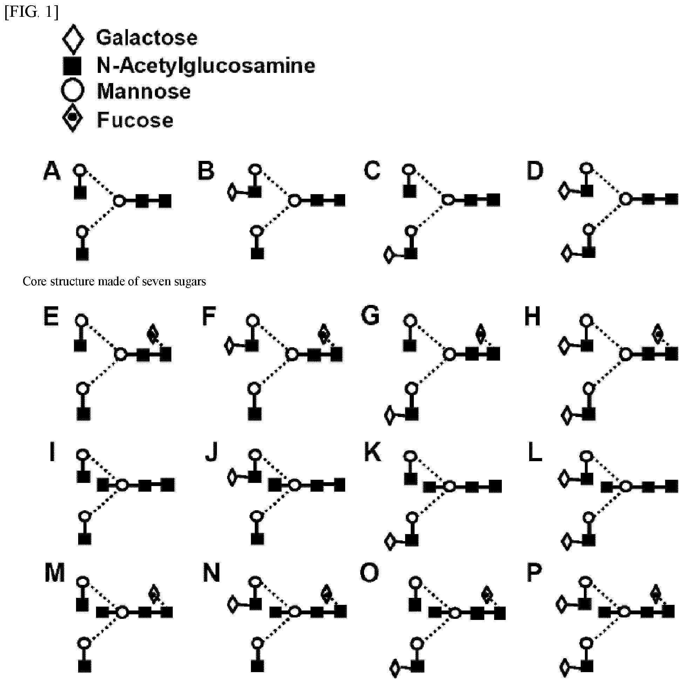

IVIG is one of a small number of treatment options for autoimmune diseases such as Kawasaki disease, idiopathic thrombocytopenic purpura, and Guillain-Barré syndrome. IVIG is produced by fractionating IgG from the plasma of several thousand healthy subjects and purifying and concentrating such. Although it is reported that the therapeutic effect is in the IgG-Fc region (non-patent literatures 1, 2) and an Asn297-binding glycan of IgG-Fc is necessary (non-patent literature 2), the mechanism of the anti-inflammatory action is unclear. The glycans of IgG-Fc are essential in expressing effector functions of IgG such as an Fcy receptor and complement activation, and removing glycans greatly impairs antibody-dependent cellular cytotoxicity (ADCC), complement-dependent cytotoxicity, and the like (non-patent literature 3). The IgG-Fc glycans have fucose, galactose, sialic acid, and the like bound to a core structure made of seven sugars and exhibit a high degree of heterogeneity (FIG. 1). It is reported that these monosaccharides bound to a nonreducing end are associated with various biological activities (non-patent literature 4).

Fucose removal is shown to augment ADCC by approximately a hundredfold (non-patent literatures 5, 6), and afucosylated monoclonal antibodies (mogamulizumab, obinutuzumab) are already seeing clinical application for adult T-cell leukemia and CD20-positive follicular lymphoma. Identifying the effective component of IVIG exhibiting an anti-inflammatory effect against autoimmune diseases and elucidating a mechanism of action thereof is also necessary in order to develop a novel antibody pharmaceutical to replace IVIG, whose demand will continue to grow.

### Prior-Art Literature

### Non-Patent Literature

Non-patent literature 1: Debre, M., et al., Infusion of Fc gamma fragments for treatment of children with acute immune thrombocytopenic purpura. Lancet, 1993. 342(8877): p. 945-9.
Non-patent literature 2: Schwab, I. and F. Nimmerjahn, Intravenous immunoglobulin therapy: how does IgG modulate the immune system? Nat Rev Immunol, 2013. 13(3): p. 176-89.
Non-patent literature 3: Mimura, Y., et al., Glycosylation engineering of therapeutic IgG antibodies: challenges for the safety, functionality and efficacy. Protein Cell, 2018. 9(1): p. 47-62.
Non-patent literature 4: Zhang, P., et al., Challenges of glycosylation analysis and control: an integrated approach to producing optimal and consistent therapeutic drugs. Drug Discov Today, 2016. 21(5): p. 740-65.
Non-patent literature 5: Shinkawa, T., et al., The absence of fucose but not the presence of galactose or bisecting N-acetylglucosamine of human IgG1 complex-type oligosaccharides shows the critical role of enhancing antibody-dependent cellular cytotoxicity. J Biol Chem, 2003. 278(5): p.3466-73.
Non-patent literature 6: Shields, R.L., et al., Lack of fucose on human IgG1 N-linked oligosaccharide improves binding to human Fcgamma RIII and antibody-dependent cellular toxicity. J Biol Chem, 2002. 277(30): p. 26733-40.

### Summary of Invention

### Problem to Be Solved by Invention

The present invention is made in view of these problems and has as an object to provide a new therapeutic drug for an anti-inflammatory disease such as an autoimmune disease.

### Means for Solving Problem

In an anti-inflammatory afucosylated immunoglobulin preparation of the present invention, an IgG antibody that is contained is made of a human serum IgG antibody in which the glycan illustrated below is bound to asparagine 297 (Asn297) of the Fc region. Here, G is galactose, N is N-acetylglucosamine, and M is mannose.

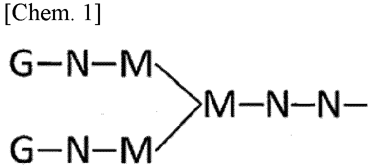

A method of producing an anti-inflammatory afucosylated immunoglobulin preparation of the present invention has: a glycan removing step of using endoglycosidase S (Endo S) on a human serum IgG antibody in order to cleave, at a chitobiose core, a glycan bound to an asparagine 297 (Asn297) residue of the Fc region and remove the glycan, excluding N-acetylglucosamine bound to the Asn297 residue and fucose bound to the N-acetylglucosamine; a fucose removing step of using an α-L-fucosidase (AlfC) to remove the fucose bound to the N-acetylglucosamine; and a transferring step of using a glycosynthase (Endo S D233Q) to transfer an oxazolinated glycan prepared from a galactosyl glycopeptide (GG-Ox) to the N-acetylglucosamine bound to the asparagine 297 (Asn297) residue of the Fc region of the human serum IgG antibody.

### Effects of Invention

The present invention can provide a new therapeutic drug for an inflammatory disease such as an autoimmune disease.

### Brief Description of Drawings

[FIG. 1] A diagram illustrating the heterogeneity of IgG-Fc glycans.
[FIG. 2] A diagram illustrating a difference in NK-cell killing action between fucosylated serum IgG (S2F), in which fucose is present in the glycans of the Fc region of the antibody, and afucosylated serum IgG (S2), in which no fucose is present in the glycans of the Fc region of the antibody.
[FIG. 3] An SDS-PAGE photographic diagram for fucosylated serum IgG (S2F), in which fucose is present in the glycans of the Fc region of the antibody, and afucosylated serum IgG (S2), in which no fucose is present in the glycans of the Fc region of the antibody.
[FIG. 4] A diagram illustrating steps by which an oxazolinated glycan (SG-Ox) is formed from a glycan isolated by Endo S from a sialylglycopeptide.
[FIG. 5] A diagram illustrating by high-performance liquid chromatography that fucose is removed in IgG (S2) of an anti-inflammatory afucosylated immunoglobulin preparation of the present example.
[FIG. 6] A diagram illustrating that normal serum IgG (95% fucosylated IgG) suppresses ADCC in a concentration-dependent manner.
[FIG. 7] A diagram illustrating that afucosylated serum IgG (S2) exhibits a significantly greater action of suppressing antibody-dependent cellular cytotoxicity (ADCC) than fucosylated serum IgG (S2F).
[FIG. 8] A photographic diagram illustrating respective SDS electrophoresis results of fucosylated serum IgG (S2F), afucosylated serum IgG (S2), and galactosylated afucosylated serum IgG (G2).
[FIG. 9] A diagram illustrating steps by which an oxazolinated glycan (GG-Ox) is formed from a glycan isolated by Endo S from a galactosyl glycopeptide.
[FIG. 10] A diagram illustrating by high-performance liquid chromatography that fucose is removed in IgG (S2) and IgG (G2) of an anti-inflammatory afucosylated immunoglobulin preparation of the present example.
[FIG. 11] A diagram illustrating that normal serum IgG (95% fucosylated IgG) suppresses ADCC in a concentration-dependent manner.
[FIG. 12] A diagram illustrating that afucosylated serum IgG (G2) exhibits a significantly greater action of suppressing antibody-dependent cellular cytotoxicity (ADCC) than fucosylated serum IgG (S2F) and afucosylated serum IgG (S2).
[FIG. 13] A diagram illustrating an anti-inflammatory effect of galactosylated afucosylated IgG (G2) on mice with collagen antibody-induced arthritis.
[FIG. 14] A diagram illustrating acute-phase protein IL-6 (A) and C-reactive protein (CRP) (B) in mice with collagen antibody-induced arthritis, the mice being administered with IgG.

### Embodiment of Invention

An embodiment of the present invention is specifically described below with reference to the included drawings. This embodiment is for facilitating understanding of the principles of the present invention. The scope of the present invention is not limited to the following embodiment, and other embodiments in which a person skilled in the art makes appropriate substitutions in the configuration of the following embodiment are also included in the scope of the present invention.

In an anti-inflammatory afucosylated immunoglobulin preparation of the present embodiment, an IgG antibody that is contained is made of a human serum IgG antibody in which the glycan illustrated below is bound to asparagine 297 (Asn297) of the Fc region. Here, S is sialic acid, G is galactose, N is N-acetylglucosamine, and M is mannose.

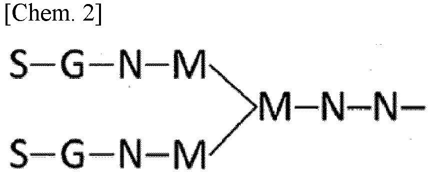

Furthermore, in the anti-inflammatory afucosylated immunoglobulin preparation of the present embodiment, an IgG antibody that is contained is made of a human serum IgG antibody in which the glycan illustrated below is bound to asparagine 297 (Asn297) of the Fc region. Here, G is galactose, N is N-acetylglucosamine, and M is mannose.

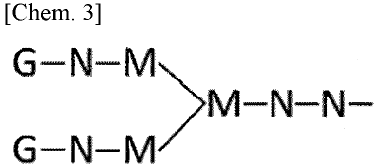

Immunoglobulins include antibodies, antibody fragments (scFv, Fab, Fc, F(ab')2), and other genetically engineered portions of antibodies but are not limited thereto. Immunoglobulins-that is, antibodies-are a group of glycoproteins present in the serum and the tissue and body fluids of all mammals. IgG (immunoglobulin G) makes up approximately 75 to 85% of the immunoglobulins in normal human serum. Glycans also play an important role in maintaining the overall three-dimensional structure of IgG. Sixteen types of glycans are present as neutralized glycans. The glycan structure of IgG is constituted by a very heterogenous mixture of glycans, but relative ratios of these sixteen types are substantially constant in healthy subjects. Note that glycans in myeloma patients and rheumatism patients are known to exhibit very specific relative ratios.

Antibody-dependent cellular cytotoxicity (ADCC) is targeted cellular cytotoxicity induced in an antibody-dependent manner by an antibody bound to a target cell binding to Fc receptors on an effector cell such as a natural killer cell (NK cell) or a macrophage. ADCC is believed to be one of the most important activities in the expression of drug efficacy in antibody pharmaceuticals.

Two N-glycoside-bound glycans are bound to the Fc region of one IgG antibody molecule. These N-glycoside-bound glycans of the antibody are complex two-chain glycans whose basic structure is a mannosyl-chitobiose core (mannosyl-chitobiose core) structure. On a nonreducing-end side, there is variety in the presence or absence of galactose and sialic acid. At a reducing end, there is variety in the presence or absence of fucose.

Removing the fucose residue from the N-acetylglucosamine of the N-glycoside-bound complex glycan reducing end bound to the antibody Fc region increases affinity with Fcy receptor IIIa. In the present specification, an afucosylated IgG having sialic acid at an end can be represented as "IgG (S2)" (or simply referred to as "afucosylated IgG" ), and an afucosylated IgG having galactose at an end can be represented as "IgG (G2)" (or simply referred to as "galactosylated afucosylated IgG" ).

IgG (S2) has a structure in which the glycan illustrated below is bound to asparagine 297 (Asn297) of the Fc region. Here, S is sialic acid, G is galactose, N is N-acetylglucosamine, and M is mannose.

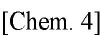

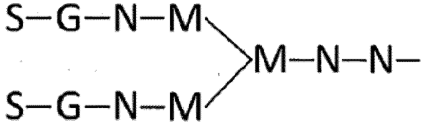

IgG (G2) has a structure in which the glycan illustrated below is bound to asparagine 297 (Asn297) of the Fc region. Here, G is galactose, N is N-acetylglucosamine, and M is mannose.

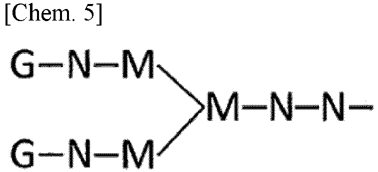

Note that the IgG antibody that is contained being made of a human serum IgG antibody in which a predetermined glycan is bound to asparagine 297 (Asn297) of the Fc region means that the human serum IgG antibody in which the predetermined glycan is bound to asparagine 297 (Asn297) of the Fc region is contained at 95% to 100%-preferably 98% to 100% and most preferably 100%-in the IgG antibody contained in the immunoglobulin preparation.

As such, the anti-inflammatory afucosylated immunoglobulin preparation in which the IgG antibody that is contained is made of a human serum IgG antibody in which the glycan illustrated below is bound to asparagine 297 (Asn297) of the Fc region (here, S being sialic acid, G being galactose, N being N-acetylglucosamine, and M being mannose ) means that the human serum IgG antibody in which the glycan illustrated below is bound to asparagine 297 (Asn297) of the Fc region is contained at 95% to 100%-preferably 98% to 100% and most preferably 100%-in the IgG antibody contained in the immunoglobulin preparation.

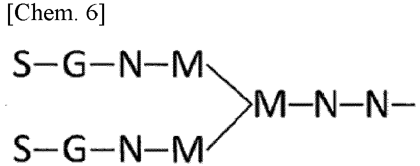

Furthermore, the anti-inflammatory afucosylated immunoglobulin preparation in which the IgG antibody that is contained is made of a human serum IgG antibody in which the glycan illustrated below is bound to asparagine 297 (Asn297) of the Fc region (here, G being galactose, N being N-acetylglucosamine, and M being mannose ) means that the human serum IgG antibody in which the glycan illustrated below is bound to asparagine 297 (Asn297) of the Fc region is contained at 95% to 100%-preferably 98% to 100% and most preferably 100%-in the IgG antibody contained in the immunoglobulin preparation.

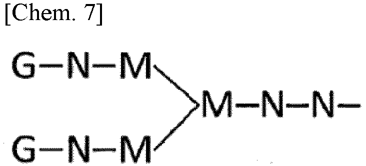

Meanwhile, fucosylated IgG can be represented as "IgG (S2F)". IgG (S2F) has a structure in which the glycan illustrated below is bound to asparagine 297 (Asn297) of the Fc region. Here, S is sialic acid, G is galactose, N is N-acetylglucosamine, M is mannose, and F is fucose.

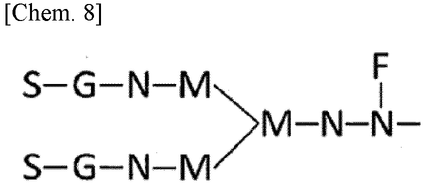

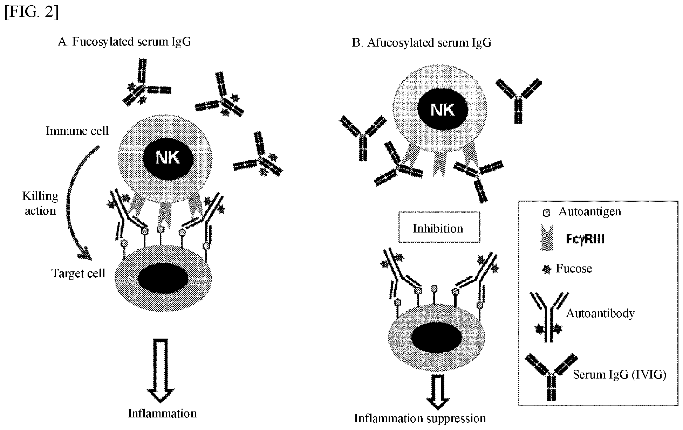

As illustrated in FIG. 2, fucosylated IgG cannot inhibit interaction between an autoantibody-antigen complex and Fcγ receptor IIIa on an NK cell. However, afucosylated IgG (the afucosylated serum IgG illustrated in B of FIG. 2 being IgG (S2) or IgG (G2) ) is believed to bind strongly to Fcγ receptor IIIa on an NK cell to inhibit the killing action of the NK cell and, as a result, suppress inflammation.

When the immunoglobulin preparation of the present invention is a liquid preparation, the preparation is used as-is or by being diluted by an appropriate solvent (for example, distilled water for injection, saline, or a glucose solution). When the preparation is a dry preparation, the preparation is used by freeze-drying the immunoglobulin solution and afterward dissolving such in an appropriate solvent (for example, distilled water for injection) at a time of use.

A route of administration of the immunoglobulin preparation of the present invention is normally injection. Intravenous administration is particularly preferable. A standard administration amount of the immunoglobulin preparation of the present invention is 50 to 1,000 mg/day of the immunoglobulin per 1 kg of bodyweight administered intravenously for one to several consecutive days. It is sufficient to increase or decrease the administration amount according to symptoms, sex, bodyweight, and the like.

The immunoglobulin solution of the present invention may contain a pharmaceutically acceptable additive (for example, a carrier, an excipient, or a diluent), stabilizer, or pharmaceutically necessary component normally used in a pharmaceutical product within a range that does not compromise the object of the present invention.

As the stabilizer, a monosaccharide such as glucose; a disaccharide such as saccharose or maltose; a sugar alcohol such as mannitol or sorbitol; a neutral salt such as sodium chloride; an amino acid such as glycine; a nonionic surfactant such as polyethylene glycol, a polyoxyethylene-polyoxypropylene copolymer (Pluronic (registered trademark)), or a polyoxyethylene sorbitan fatty-acid ester (Tween); and the like are given as examples. The stabilizer is preferably added at approximately 1 to 10 w/v%.

A disease for which the immunoglobulin preparation of the present invention is administered is not particularly limited. However, for example, Kawasaki disease, idiopathic thrombocytopenic purpura, Guillain-Barré syndrome, multiple sclerosis, chronic rheumatoid arthritis, systemic lupus erythematosus, pemphigus, bullous pemphigoid, myasthenia gravis, ANCA-associated vasculitis, chronic inflammatory demyelinating polyneuropathy, autoimmune neutropenia, autoimmune hemolytic anemia, autoimmune acquired factor VIII deficiency, stiff-person syndrome, multifocal neuropathy, Behçet's disease, ulcerative colitis, Crohn's disease, Reiter's syndrome, polymyositis, dermatomyositis, circumscribed scleroderma, systemic scleroderma, Sjögren syndrome, anti-glomerular basement membrane disease, primary sclerosing cholangitis, antiphospholipid antibody syndrome, toxic epidermal necrolysis, graft-versus-host disease, and sepsis can be mentioned.

Note that antibody pharmaceuticals in which fucose is removed from the glycans of the Fc region of a monoclonal IgG antibody are developed for an object of augmenting antibody-dependent cellular cytotoxicity (ADCC) (e.g.: mogamulizumab (product name: Poteligeo^{™})). Conventional afucosylated antibodies are antigen-specific monoclonal antibodies produced from a mammalian host cell having a manipulated glycan-related gene. These antibodies are adapted for malignant tumors. In contrast, the immunoglobulin of the invention of the present application enzymatically removes fucose of a nonspecific IgG antibody derived from serum and afterward transfers homogenous glycans by a chemical enzyme reaction. This immunoglobulin is adapted for an autoimmune disease. That is, the immunoglobulin preparation of the invention of the present application is used for an object of inhibiting an undesirable immune response by an autoantibody.

A method of producing the anti-inflammatory afucosylated immunoglobulin preparation of the present embodiment has: a glycan removing step of cleaving, at a chitobiose core, a glycan bound to an asparagine 297 (Asn297) residue of the Fc region of a human serum IgG antibody and removing the glycan, excluding N-acetylglucosamine bound to the Asn297 residue and fucose bound to the N-acetylglucosamine; a fucose removing step of removing the fucose bound to the N-acetylglucosamine; and a transferring step of using a glycosynthase (Endo S D233Q) to transfer an oxazolinated glycan prepared from a yolk-derived sialylglycopeptide (SG-Ox) to the N-acetylglucosamine bound to the asparagine 297 (Asn297) residue of the Fc region of the human serum IgG antibody.

A method of producing an anti-inflammatory afucosylated immunoglobulin preparation of the present embodiment has: a glycan removing step of using endoglycosidase S (Endo S) on a human serum IgG antibody in order to cleave, at a chitobiose core, a glycan bound to an asparagine 297 (Asn297) residue of the Fc region and remove the glycan, excluding N-acetylglucosamine bound to the Asn297 residue and fucose bound to the N-acetylglucosamine; a fucose removing step of using an α-L-fucosidase (AlfC) to remove the fucose bound to the N-acetylglucosamine; and a transferring step of using a glycosynthase (Endo S D233Q) to transfer an oxazolinated glycan prepared from a yolk-derived galactosyl glycopeptide (GG-Ox) to the N-acetylglucosamine bound to the asparagine 297 (Asn297) residue of the Fc region of the human serum IgG antibody.

The enzyme that cleaves the glycan bound to the asparagine 297 (Asn297) residue of the Fc region at the chitobiose core is endoglycosidase S (Endo S).

The enzyme that removes the fucose bound to N-acetylglucosamine is an α-L-fucosidase (AlfC). As the α-L-fucosidase, there is 1,2-α-L-fucosidase, 1,3-α-L-fucosidase, or 1,6-α-L-fucosidase. Here, it is preferable to use 1,6-α-L-fucosidase.

The oxazolinated glycan (SG-Ox or GG-Ox) is prepared by subjecting a reducing end of sialylglycan to dehydration condensation.

The enzyme that transfers SG-Ox or GG-Ox to the glycan-removed IgG is a glycosynthase (Endo S D233Q).

### Examples

### 1. Production of anti-inflammatory afucosylated immunoglobulin preparation

### 1a-1. Purification of serum-derived IgG

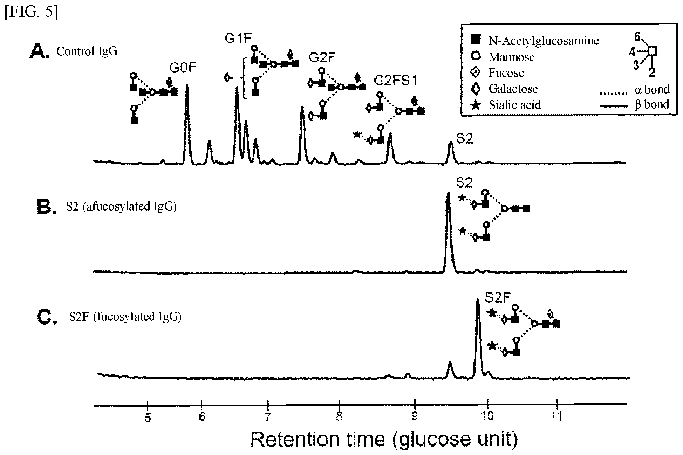

20 mL of serum prepared from the blood of a healthy subject was dialyzed using a 0.01 M phosphate buffer solution (pH 7.0) and afterward added to a diethylaminoethyl (DEAE) cellulose anion exchange column equilibrated using the same buffer solution (DE52, Whatman Biosystems, Chalfont, St. Giles, UK) (1 × 30 cm) to collect IgG included in a flow-through fraction. A glycan structure of the serum IgG was analyzed by high-performance liquid chromatography as described below (FIG. 5-A), and it was confirmed that 95% or more was fucosylated.

### 1a-2. Deglycosylation of serum IgG

Endo S was used as the endoglycosidase that cleaves the Fc-bound glycan of IgG. Hydrolysis was performed between two N-acetylglucosamines of the chitobiose core of the glycan, and deglycosylation was performed, leaving the two sugars N-acetylglucosamine and fucose. The deglycosylated IgG was purified using Protein G Sepharose 4 (GE).

### 1a-3. Defucosylation of serum IgG

An α-L-fucosidase (AlfC) was used to remove fucose. The DNA sequence of the expression vector of the present enzyme has been reported (sequence no. 1) and was expressed according to the literature in BL21(DE3) *E. coli.* AlfC was reacted overnight at 37°C with the IgG deglycosylated by Endo S in 50 mM tris-HCl (pH 7.4) to remove fucose.

### 1a-4. Formation of sialylglycan oxazoline (SG-Ox)

The glycans of the sialylglycopeptide illustrated below (10 mg, TCI) were cleaved using Endo S in a 50 mM phosphate buffer solution (pH 6.0). 2-Chloro-1,3-dimethylimidazolinium chloride (TCI) and triethylamine were added. This was left standing for 1 hour at 0°C, and SG-Ox was prepared by a dehydration condensation reaction of a reducing end.

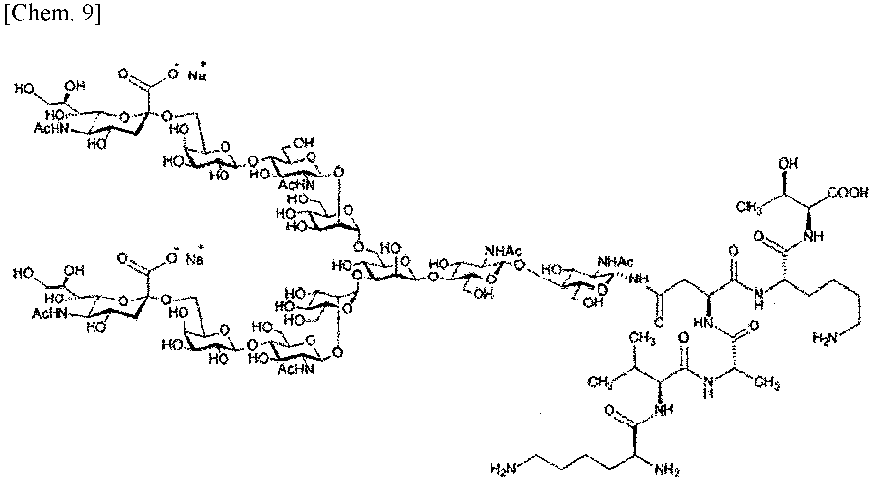

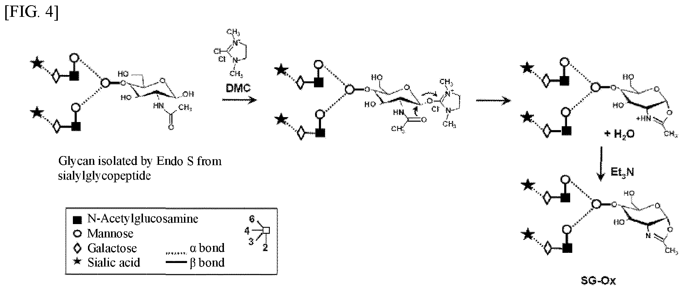

FIG. 4 illustrates the steps of forming the oxazolinated glycan (SG-Ox) from the sialyl glycan isolated by Endo S. The structure of SG-Ox is illustrated below.

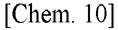

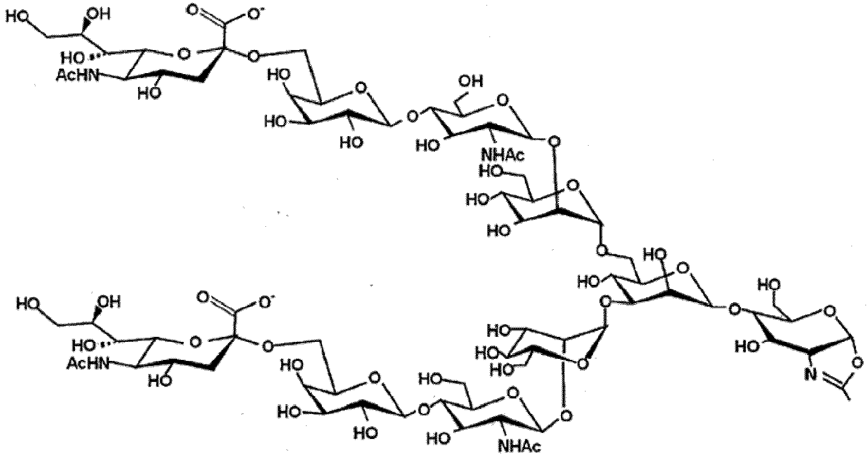

### 1a-5. Making of afucosylated IgG

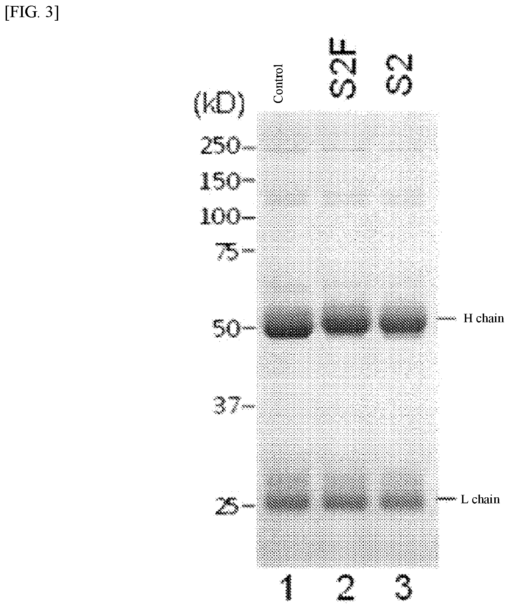

The DNA sequence of the expression vector of the glycosynthase Endo S D233Q has been reported in literature (sequence no. 2) and was expressed in BL21(DE3) *E. coli.* The glycan transferring reaction was performed using SG-Ox by incubating for 3 hours at 30°C in 50 mM tris-HCl (pH 7.4). This produced the anti-inflammatory afucosylated immunoglobulin preparation of the present example. This afucosylated IgG of the present example can be represented as "IgG (S2)". It was confirmed that both IgG (S2) and IgG (S2F), the fucosylated IgG thereof, had a purity of 95% or more in SDS-PAGE (FIG. 3). The glycan transferring reaction of SG-Ox to GlcNAc-IgG is illustrated below.

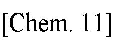

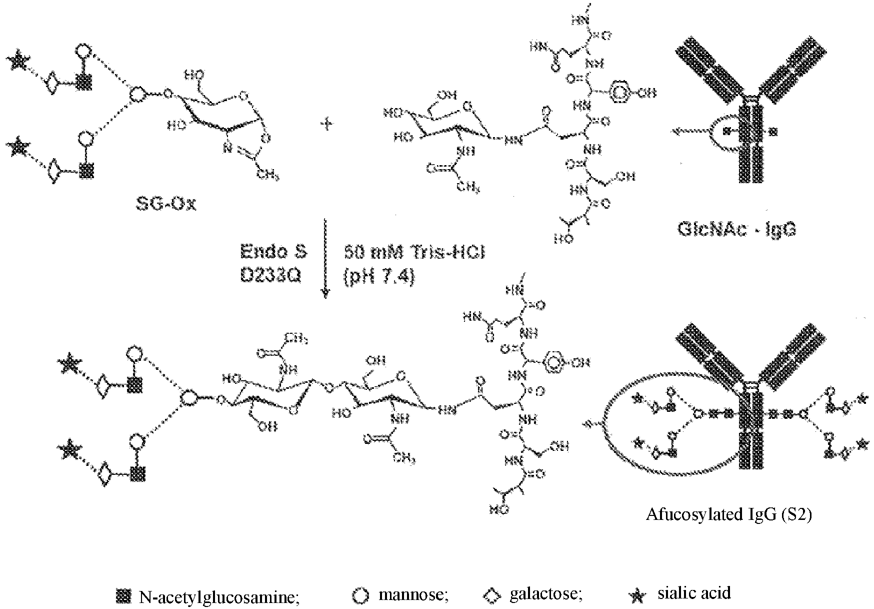

To confirm glycan modification in the anti-inflammatory afucosylated immunoglobulin preparation of the present example, IgG (S2) and IgG (S2F) in which the glycans are replaced were subjected to N-glycosidase F digestion to isolate the glycans. Afterward, fluorescent labeling was performed using 2-aminobenzamide, and analysis was performed using high-performance liquid chromatography (HPLC). Note that IgG (S2F) is a fucosylated IgG of a comparative example. Specifically, it is a human serum IgG in which the glycan illustrated below is bound to asparagine 297 (Asn297) of the Fc region.

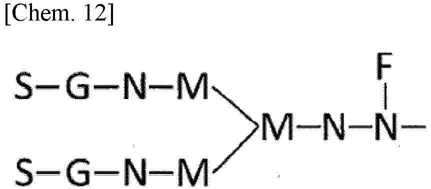

As illustrated in FIG. 5, the glycans of the control IgG substantially have fucose. However, it can be confirmed that fucose is removed in IgG (S2) of the anti-inflammatory afucosylated immunoglobulin preparation of the present example.

### 1b. ADCC suppression (anti-inflammatory) effect by afucosylated serum IgG

An anti-inflammatory action of the anti-inflammatory afucosylated immunoglobulin preparation of the present example was confirmed by the following method.

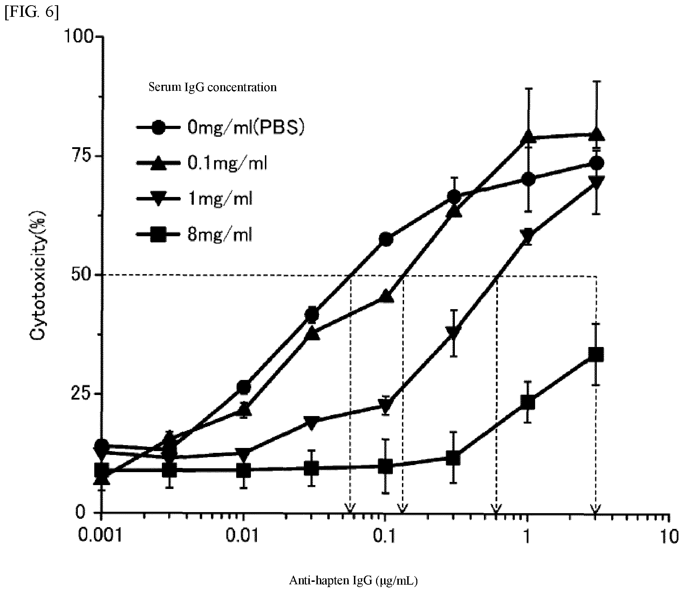

As a preliminary test, an effect of normal serum IgG (95% fucosylated IgG; the control IgG illustrated in A in FIG. 5) on ADCC was examined using an ADCC reporter bioassay kit (Promega). Target cells were labeled using a hapten (4-hydroxy-3-iodo-5-nitrophenacetyl) and sensitized by an anti-hapten monoclonal IgG antibody (0.001 to 3 µg/mL) (FIG. 6).

A magnitude of ADCC of the anti-hapten IgG is indicated by an anti-hapten IgG concentration that can induce target-cell death (cytotoxicity; FIG. 6, Y axis) of 50% of the maximum level (EC50). The lower the EC50, the higher the ADCC of the specific antibody can be interpreted to be. Upon adding serum IgG at concentrations of 0.01, 0.1, 1, and 8 mg/mL to the ADCC measurement system to compare EC50 of the anti-hapten IgG, it was shown that the higher the concentration of the added serum IgG, the more the EC50 rose (shifted to the right on the X axis) and the more ADCC was suppressed (FIG. 6).

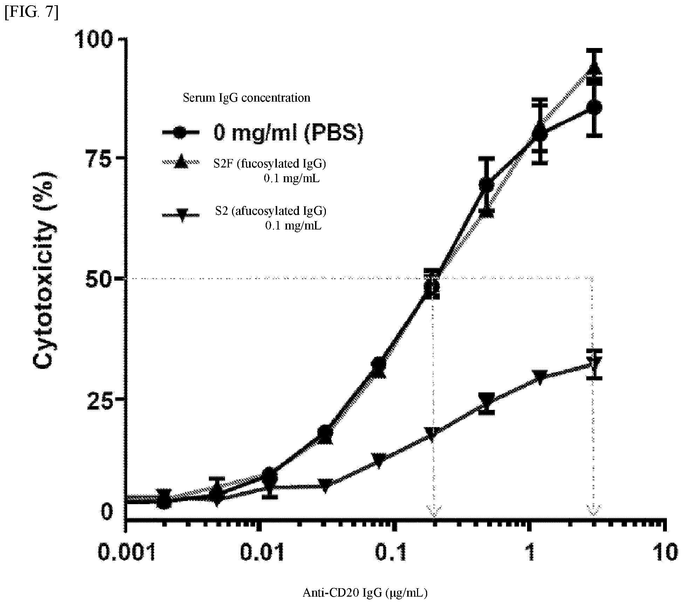

Meanwhile, to examine whether there is a difference in ADCC suppression action between afucosylated IgG (S2) that is a glycan-modified serum IgG and fucosylated IgG (S2F), ADCC of anti-CD20 IgG in the presence of such were compared (FIG. 7). The S2 and S2F concentrations were made to be 0.1 mg/mL. For S2F, the EC50 was the same as the control (0.2 µg/mL), and no suppressing action was exhibited (FIG. 7). Meanwhile, in the presence of S2, the EC50 was > 3 µg/mL, a concentration no less than 15 times higher (FIG. 7). That is, it can be said that S2 exhibited an ADCC suppressing action no less than 15 times that of S2F. Therefore, an anti-inflammatory effect can be expected by an administration amount less than normal IVIG if afucosylated IVIG is used. Moreover, there is a possibility of an anti-inflammatory effect being exhibited even for a case in which normal IVIG is ineffective.

### 2. Preparation of anti-inflammatory galactosylated afucosylated immunoglobulin preparation

### 2a-1. Purification of serum-derived IgG

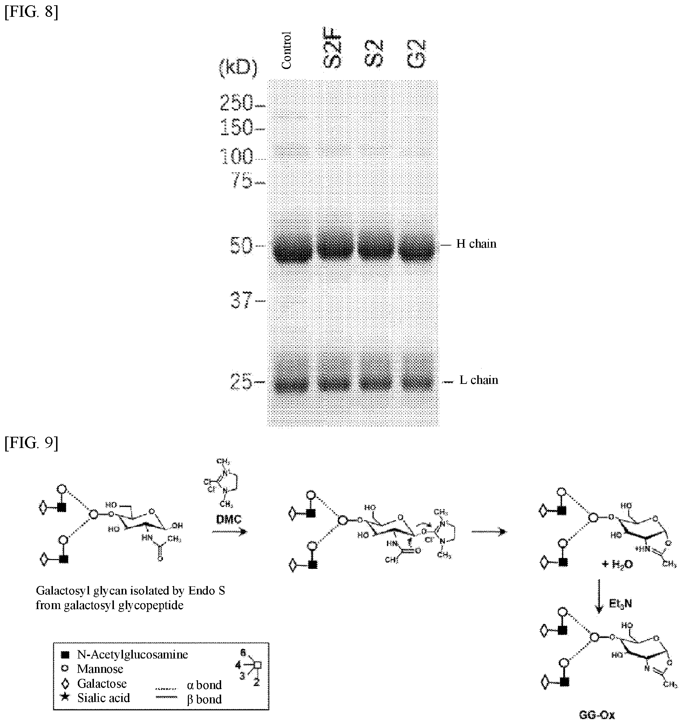

20 mL of serum prepared from the blood of a healthy subject was dialyzed using a 0.01 M phosphate buffer solution (pH 7.0) and afterward added to a diethylaminoethyl (DEAE) cellulose anion exchange column equilibrated using the same buffer solution (DE52, Whatman Biosystems, Chalfont, St. Giles, UK) (1 × 30 cm) to collect IgG included in a flow-through fraction. The purity was confirmed to be 95% or more in SDS-PAGE (FIG. 8, lane 1).

### 2a-2. Glycan cleaving of serum IgG

Endo S was used as the endoglycosidase that cleaves the Fc-bound glycan of IgG. The DNA sequence of the expression vector of Endo S has been reported and was expressed in BL21(DE3) *E. coli.* Using Endo S immobilized to cyanogen bromide-activated Sepharose 4B beads, hydrolysis was performed between two N-acetylglucosamines of the chitobiose core of the glycan, and deglycosylation was performed, leaving the two sugars N-acetylglucosamine and fucose. The deglycosylated IgG was purified using Protein G Sepharose 4 (GE).

### 2a-3. Defucosylation of serum IgG

An α-L-fucosidase (AlfC) was used to remove fucose. The DNA sequence of the expression vector of the present enzyme has been reported (sequence no. 1) and was expressed according to the literature in BL21(DE3) *E. coli.* AlfC was reacted overnight at 37°C with the IgG deglycosylated by Endo S in 50 mM tris-HCl (pH 7.4) to remove fucose.

### 2a-4. Formation of galactosyl glycan oxazoline (GG-Ox)

A sialylglycopeptide (10 mg, TCI) was reacted overnight at 37°C with a sialidase (Roche) and Sepharose-immobilized Endo S in a 50 mM phosphate buffer solution (pH 6.0). The galactosyl glycopeptide illustrated below was the intermediate body. The glycans thereof were cleaved, and a galactosyl glycan was formed. Moreover, 2-chloro-1,3-dimethylimidazolinium chloride (TCI) and triethylamine were added. This was left standing for 1 hour at 0°C, and GG-Ox was prepared by a dehydration condensation reaction of a reducing end. The GG-Ox was purified by a cellulose column (Sigma-Aldrich). Note that in the above, the galactosyl glycopeptide was obtained by desialylation in which a sialidase is used on a sialylglycopeptide. However, desialylation is possible without using an enzyme. For example, the galactosyl glycopeptide can also be obtained by desialylation in which the sialylglycopeptide is heat-treated at a pH of 1 to 2.

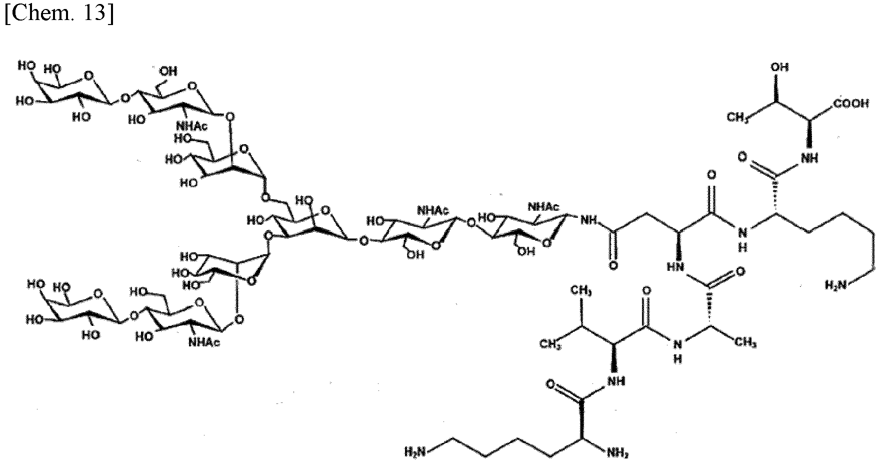

FIG. 9 illustrates steps of forming GG-Ox from a glycan isolated by Endo S. The structure of GG-Ox is illustrated below.

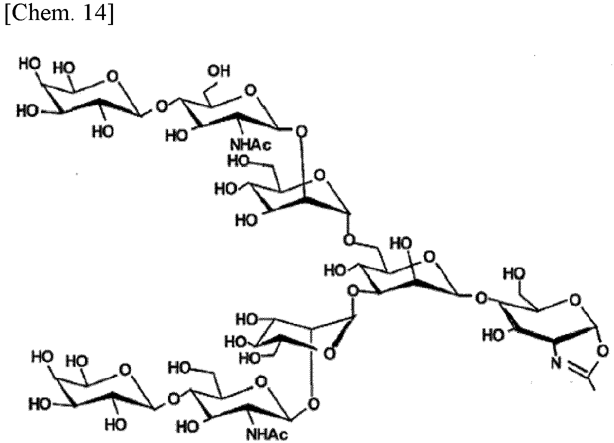

### 2a-5. Making of galactosylated afucosylated IgG

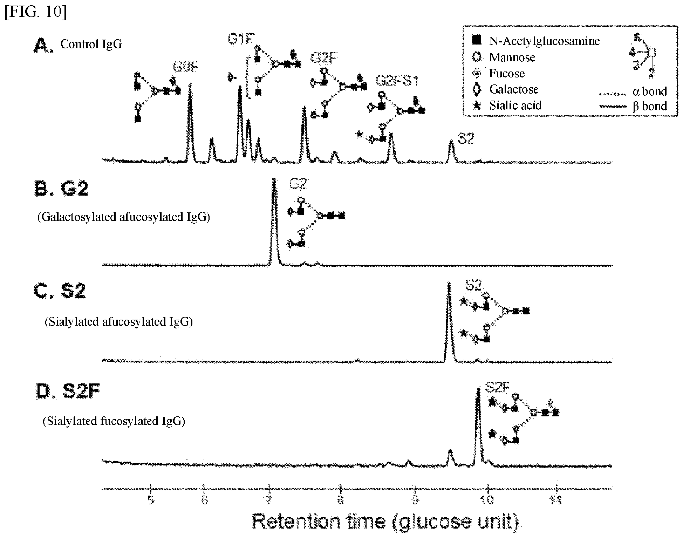

The DNA sequence of the expression vector of the glycosynthase Endo S D233Q has been reported in literature and was expressed in BL21(DE3) *E. coli.* The glycan transferring reaction was performed using GG-Ox by incubating for 4 hours at 30°C in Endo S D233Q and 50 mM tris-HCl (pH 7.4). This produced the anti-inflammatory galactosylated afucosylated immunoglobulin preparation of the present example. This galactosylated afucosylated IgG of the present example can be represented as "IgG (G2)". To confirm glycan modification, IgG (S2, S2F, G2) in which the glycans are replaced were subjected to N-glycosidase F digestion to isolate the glycans. Afterward, fluorescent labeling was performed using 2-aminobenzamide, and analysis was performed using high-performance liquid chromatography (HPLC) (FIG. 10). The glycan transferring reaction of GG-Ox to GlcNAc-IgG is illustrated below.

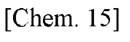

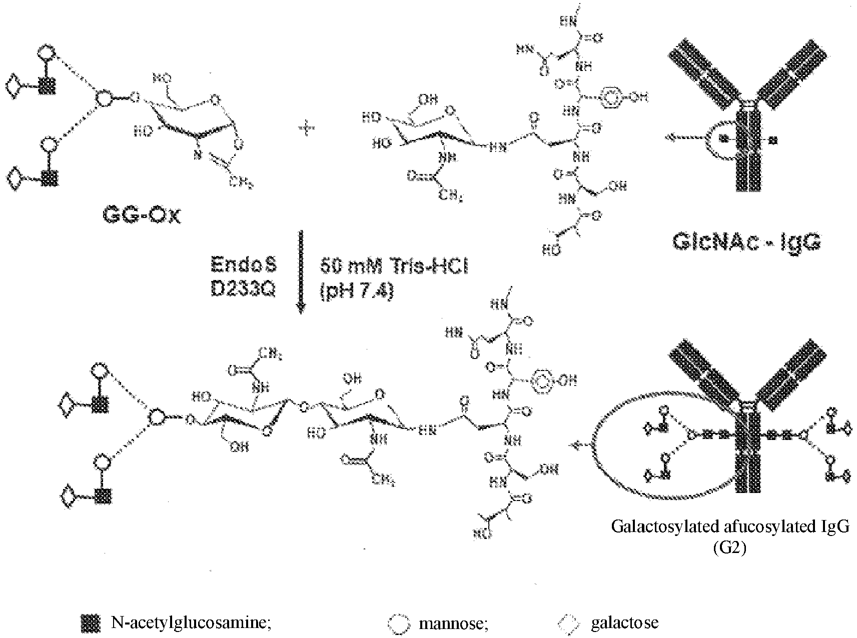

### 2b. ADCC suppression (anti-inflammatory) effect of galactosylated afucosylated immunoglobulin

### 2b-1. In vitro ADCC suppression (anti-inflammatory) effect

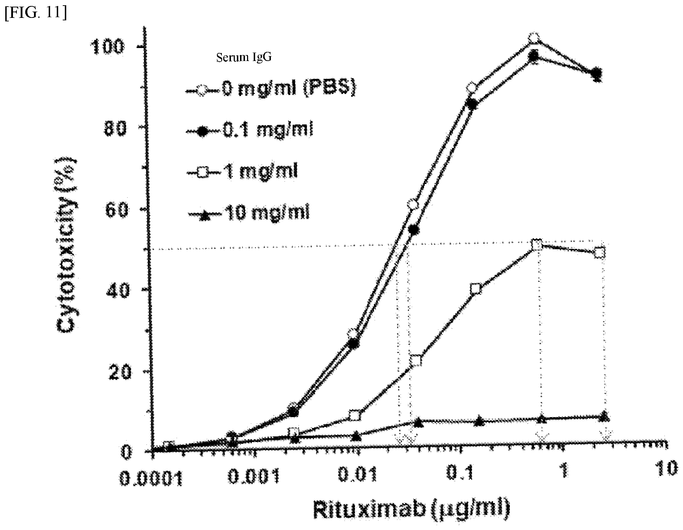

It was found by the following method that serum IgG removed of fucose has an anti-inflammatory action and that having galactose at an end augments this action. As a preliminary test, an effect of the presence of normal serum IgG (IVIG, 95% fucosylated; FIG. 10A) on ADCC was examined using an ADCC reporter bioassay kit (Promega). The target cells (Raji cells) were sensitized by a CD20 antibody (rituximab) (0.001 to 3 µg/mL; FIG. 11). A magnitude of ADCC is indicated by an antibody concentration that can induce target-cell death (cytotoxicity; FIG. 11 Y axis) of 50% of the maximum level (EC50; FIG. 11 X axis). It can be interpreted that the smaller the EC50 value, the greater the ADCC. Upon adding serum IgG (IVIG) to the ADCC measurement system at concentrations of 0, 0.1, 1, and 10 mg/mL to compare EC50 values, it was shown that these were respectively 0.026, 0.032, 0.6, and > 2 µg/mL; the higher the IVIG concentration, the more the EC50 value rose and the more ADCC was suppressed (FIG. 11).

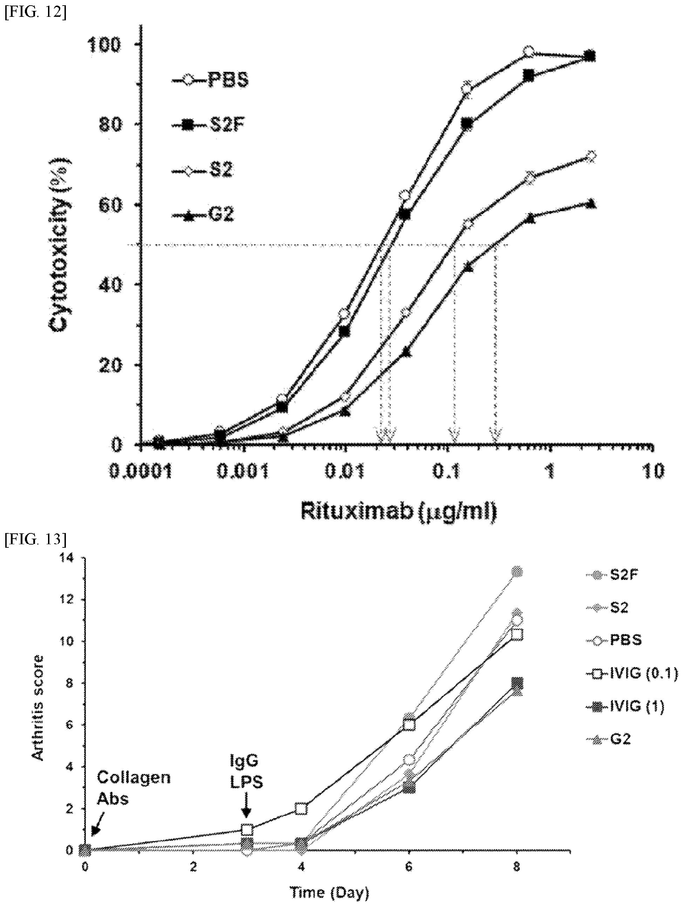

Next, ADCC was measured in the presence of glycan-modified afucosylated IgG (S2), galactosylated afucosylated IgG (G2), agalactosylated afucosylated IgG (G0), and fucosylated IgG (S2F) at a concentration of 0.1 mg/mL (FIG. 12). The EC50 values were respectively 0.023, 0.028, 0.08, 0.12, and 0.3 µg/mL in the presence of PBS (negative control), S2F, G0, S2, and G2. The strongest inhibitory action was exhibited in the presence of G2 (FIG. 12) This corresponds to relative ADCC being 100%, 82%, 29%, 19%, and 7.6%. That is, it can be said that G2 exhibited an ADCC suppressing action that was respectively 11 times and 2.5 times greater than S2F and S2. Therefore, by using galactosylated afucosylated IVIG, an anti-inflammatory effect can be expected from a smaller administration amount than normal IVIG. Moreover, Fcy receptor IIIa has two allotypes, and Fcy receptor IIIa including valine 158 (Val158) has higher affinity to IgG and higher ADCC than Fcy receptor IIIa including phenylalanine 158 (Phe158). The present results were observed in effector cells expressing Fcy receptor IIIa-Val158. However, a similarly strong ADCC inhibiting action was seen with Fcy receptor IIIa-Phe158. Therefore, G2 is effective in patients of both allotypes, and a strong anti-inflammatory action can be expected to be exhibited even in a patient for whom normal IVIG has no effect.

### 2b-2. In vivo ADCC suppression (anti-inflammatory) effect

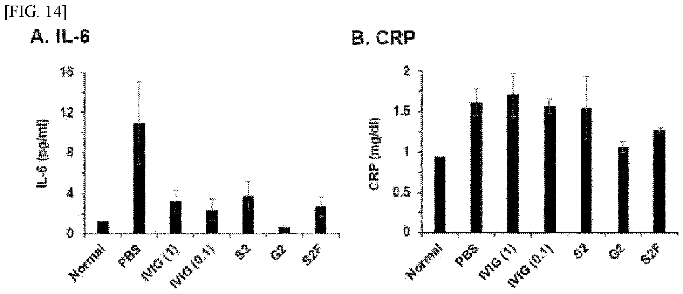

Collagen antibody-induced arthritis (collagen antibody-induced arthritis (CAIA)) model mice were used to examine whether afucosylated IgG having an ADCC suppressing action (S2, G2) (FIG. 12) exhibit an anti-inflammatory action even in vivo. On day 0, a collagen-antibody cocktail (Chondrex Inc., cat. #53010) was intravenously administered to DBA/1J mice (female, 8 weeks old; Japan SLC) (1.5 mg/mouse). On day 3, a lipopolysaccharide (LPS; 12.5 µg/mouse) was intraperitoneally administered to induce arthritis. For treatment, IgG (S2, S2F, G2; FIGS. 8, 10) was intravenously administered 1 hour prior to LPS administration (0.1 g / kg bodyweight, n = 3/group). Serum IgG (IVIG) was administered in two dosage groups-a low-dose group (0.1 g / kg bodyweight) and a high-dose group (1 g / kg bodyweight). The group administered with a high dose of IVIG is a positive control; this is a dose used to treat inflammatory disease in humans. As a negative control, a phosphate buffer solution (PBS, 0.33 mL) was used. On days 3, 4, 6, and 8, a severity of the arthritis was evaluated on a five-step scale (0: no change; 1: swelling of toes; 2: swelling of toes and soles; 3: swelling of entire foot; 4: severe swelling). This was represented by a total score for the four limbs. On day 8, the group administered with G2 and the group administered with a high dose of IVIG had similarly low arthritis scores (FIG. 13). Meanwhile, the groups administered with S2, S2F, and a low dose of IVIG had high arthritis scores like the group administered with PBS. On day 8, blood was collected by cardiac puncture under isoflurane anesthesia, and the inflammatory cytokine interleukin 6 (IL-6) and C-reactive protein (CRP), which are acute-phase proteins, were measured. The PBS group had the highest serum IL-6 concentration, at 10.9 pg/mL. The healthy (normal) group and the group administered with G2 had low values, at 1.3 pg/mL and 0.7 pg/mL, respectively (FIG. 14A). Meanwhile, the groups administered with S2, S2F, and IVIG had values 2 to 3 times higher than that of the healthy group. For a serum CRP value as well, the group administered with G2 had a low value like the healthy group (FIG. 14B). As above, it was able to be confirmed that G2 exhibits a remarkable anti-inflammatory action even in vivo.

### Industrial Applicability

The present invention can be used to treat an autoimmune disease.

### Sequence-Listing Free Text

Sequence no. 1, 2: enzymes

## Claims

1. An anti-inflammatory afucosylated immunoglobulin preparation, wherein an IgG antibody that is contained is made of a human serum IgG antibody in which the glycan illustrated below is bound to asparagine 297 (Asn297) of the Fc region (here, G being galactose, N being N-acetylglucosamine, and M being mannose ).

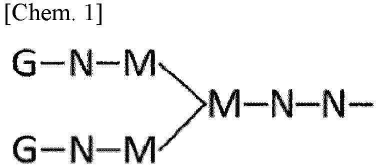

2. The anti-inflammatory afucosylated immunoglobulin preparation of claim 1, wherein the preparation is used to prevent and/or treat an autoimmune disease.

3. The anti-inflammatory afucosylated immunoglobulin preparation of claim 2, wherein the autoimmune disease is any among Kawasaki disease, idiopathic thrombocytopenic purpura, Guillain-Barré syndrome, chronic inflammatory demyelinating polyneuropathy, autoimmune neutropenia, autoimmune hemolytic anemia, autoimmune acquired factor VIII deficiency, multiple sclerosis, myasthenia gravis, stiff-person syndrome, multifocal neuropathy, ANCA-associated vasculitis, chronic rheumatoid arthritis, systemic lupus erythematosus, pemphigus, bullous pemphigoid, Behçet's disease, ulcerative colitis, Crohn's disease, Reiter's syndrome, polymyositis, dermatomyositis, circumscribed scleroderma, systemic scleroderma, Sjögren syndrome, anti-glomerular basement membrane disease, primary sclerosing cholangitis, antiphospholipid antibody syndrome, toxic epidermal necrolysis, graft-versus-host disease, and sepsis.

4. A method of producing an anti-inflammatory afucosylated immunoglobulin preparation, comprising: a glycan removing step of using endoglycosidase S (Endo S) on a human serum IgG antibody in order to cleave, at a chitobiose core, a glycan bound to an asparagine 297 (Asn297) residue of the Fc region and remove the glycan, excluding N-acetylglucosamine bound to the Asn297 residue and fucose bound to the N-acetylglucosamine;
a fucose removing step of using an α-L-fucosidase (AlfC) to remove the fucose bound to the N-acetylglucosamine; and
a transferring step of using a glycosynthase (Endo S D233Q) to transfer an oxazolinated glycan prepared from a galactosyl glycopeptide (GG-Ox) to the N-acetylglucosamine bound to the asparagine 297 (Asn297) residue of the Fc region of the human serum IgG antibody.

5. The method of producing an anti-inflammatory afucosylated immunoglobulin preparation of claim 4, wherein in the fucose removing step, the α-L-fucosidase is 1,6-α-L-fucosidase.
